# EUROPEAN PATENT SPECIFICATION

(11) **EP 4 074 306 B1**
(45) Date of publication and mention of the grant of the patent: **04.11.2026**
(21) Application number: 22177066.2
(22) Date of filing: 20.09.2018
(51) Int. Cl.: A61K 9/10, A61K 9/00, A61K 31/395, A61K 31/165, A61K 47/10, A61K 47/60, A61P 27/06, A61P 35/00, A61P 31/00

(54) **OPHTHALMIC PHARMACEUTICAL COMPOSITION, OPHTHALMIC KIT, AND MEDICAL APPLICATION THEREOF**
OPHTHALMISCHE PHARMAZEUTISCHE ZUSAMMENSETZUNG, OPHTHALMISCHES KIT UND MEDIZINISCHE ANWENDUNG DAVON
COMPOSITION PHARMACEUTIQUE OPHTALMIQUE, KIT OPHTALMIQUE ET SON APPLICATION MÉDICALE

(30) Priority: 22.09.2017 CN 201710863869
(43) Date of publication of application: 19.10.2022
(62) Document number(s) of the earlier application(s) in accordance with Art. 76 EPC: 18859935.1 / 3 685 828
(73) Proprietor: Shenyang Xingqi Pharmaceutical Co., Ltd., Shenyang, Liaoning 110167 (CN)
(72) Inventor: YANG, Qiang, Shenyang, 110163 (CN); LIU, Jidong, Shenyang, 110163 (CN)
(74) Representative: Lavoix

(56) References cited:
- WO-A1-2006/093758
- WO-A1-98/32421
- CN-A- 101 234 084
- CN-A- 104 398 473
- US-A1- 2006 009 498
- US-A1- 2012 022 137
- FABIENNE DANHIER ET AL: "PLGA-based nanoparticles: An overview of biomedical applications", JOURNAL OF CONTROLLED RELEASE, vol. 161, no. 2, 1 July 2012 (2012-07-01), AMSTERDAM, NL, pages 505 - 522, XP055561461, ISSN: 0168-3659, DOI: 10.1016/j.jconrel.2012.01.043

## Description

### Technical Field

The invention belongs to the field of medicine, specifically relates to an ophthalmic pharmaceutical composition.

### Background Art

Anatomically, the eyeball includes two parts, anterior and posterior segments of eyeball, wherein the anterior segment of eyeball accounts for approximately 1/3 and is composed of cornea, pupil, iris, aqueous humor, ciliary body and lens, while the posterior segment of eyeball accounts for approximately 2/3 and is composed of vitreum, retina, macula lutea, optic nerve, choroid and sclera. Abnormalities in any part of the eyeball may cause eye diseases, wherein eye diseases such as retinal phlebitis, diabetic retinopathy, proliferative vitreoretinopathy, choroidal neovascularization, age-related macular degeneration, macular cystoid edema, vitreous macular adhesion, macular hole are the main causes of visual impairments. At present, ocular surface administration and systemic administration are still the main modes of administration for the treatment of diseases in the posterior segment of eyeball. However, due to the presence of tear barrier, corneal barrier, and conjunctival barrier, the ocular surface administration is difficult to reach the posterior segment of eyeball; in addition, due to the effects of blood-aqueous humor and blood-retina barriers, the systemic administration requires large dose and multiple administrations in order to make the amount of drug reaching the posterior segment of eyeball meet the therapeutic need. Based on this situation, in order to increase the amount of drug reaching the posterior segment of eyeball, periocular injection and intravitreal injection are gradually adopted; however, most drugs have a short half-life in vitreum, which requires multiple injections to maintain the amount of drug in vitreum to meet the treatment requirements , which causes pain to patients, reduces patient compliance, and increases the risk of intraocular infections.

In order to solve the problems caused by multiple injections, sustained-release implants have been clinically used to treat diseases in the posterior segment of eyeball. The sustained-release implants are classified into non-biodegradable and biodegradable types according to materials. For example, Vitrasert, a ganciclovir implant developed by Australian company pSivida for the treatment of cytomegalovirus retinitis and approved by the FDA in 1996, uses non-biodegradable materials polyvinyl alcohol and ethylene-vinyl acetate copolymer as release-controlling material. In 2005, Retisert, a fluocinonide implant developed by the same company mainly for the treatment of non-infective uveitis was approved by the FDA. Retisert uses non-biodegradable materials polyvinyl alcohol and silicone oil as release-controlling materials. Since non-biodegradable sustained-release implants cannot be degraded, they need to be removed through secondary surgery. In order to solve this problem, biodegradable implants are developed. Commonly used biodegradable sustained-release materials include hypromellose, polylactic acid, polyglycolic acid, and polylactic acid-co-glycolic acid copolymer, and representative products in the market are Surodex and Ozurdex of Allergancompany, in which the former uses polylactic acid-co-glycolic acid copolymer and hypromellose as materials to prepare a dexamethasone implant, and can continuously release dexamethasone for 7 to 10 days for the treatment of anti-inflammatory after cataract surgery, while the latter uses polylactic acid-co-glycolic acid copolymer as material to prepare a dexamethasone implant for the treatment of macular edema and uveitis. However, both of the non-biodegradable implants and the biodegradable implants currently need to be implanted into the eye through surgery or special drug delivery devices (for example, Ozurdex is equipped with a special injection device). The operation is more complicated and brings some pains to patients. Patent document US 2006/0009498 A1 discloses an ophthalmic composition comprising a corticosteroid component, a biocompatible polymeric component and an ophthalmically compatible solvent.

There is currently a need for an ophthalmic drug that is simple in administration and has a sustained-release effect.

### Contents of the Invention

The present invention provides an ophthalmic pharmaceutical composition, which can be injected into an eye by injection. The ophthalmic pharmaceutical composition of the present invention can slowly release an active pharmaceutical ingredient in the eye for a long time and can be degraded in the eye.

The present invention relates in a first aspect to an ophthalmic sustained-release drug delivery system, consisting of the following components: a solid matrix having a biocompatibility, a liquid matrix having a biocompatibility, an active pharmaceutical ingredient; wherein, the solid matrix is polylactic acid-co-glycolic acid copolymer; the liquid matrix is polyethylene glycol, the active pharmaceutical ingredient is at least one selected from the group consisting of dexamethasone, latanoprost, dasatinib, triamcinolone, and pharmaceutically acceptable salts thereof, the weight ratio of the active pharmaceutical ingredient, the liquid matrix, and the polylactic acid-co-glycolic acid copolymer is 1:(1-2):(0.1-2).

In some embodiments of the first aspect of the present invention, the polylactic acid-co-glycolic acid copolymer has a weight average molecular weight of 1,000 to 500,000, for example, 5,000 to 50,000, 3000, 4000, 6000, 8000, 9000, 10,000, 40,000, 60,000, 80,000, 100,000, 200,000, 300,000, 400,000, and 450,000.

In the present invention, the number average molecular weight is measured according to a conventional method, such as an end-group analysis method, a boiling point elevation method, a freezing point reduction method, a vapor pressure osmosis method, and a thin film osmosis method.

In the present invention, the weight average molecular weight is measured according to a conventional method, such as a light scattering method, a gel chromatography method, an ultracentrifugal sedimentation rate method, and a small-angle X-ray diffraction method.

In the first aspect of the present invention, the active pharmaceutical ingredient is selected from the group consisting of dexamethasone, latanoprost, dasatinib, triamcinolone, and pharmaceutically acceptable salts thereof.

In the first aspect of the present invention, the ophthalmic pharmaceutical composition is an ophthalmic pharmaceutical preparation, preferably an ophthalmic pharmaceutical injection preparation.

A second aspect, which is not claimed, relates to a method for preparing the ophthalmic pharmaceutical composition according to the first aspect of the present invention, comprising the following steps:
(1) mixing the solid matrix with the liquid matrix to obtain a mixed substance;
(2) mixing the mixed substance obtained in step (1) with the active pharmaceutical ingredient.

In some embodiments of the second aspect of the present invention, in step (1), the mixing is performed at a temperature of 20 °C to 100 °C, such as 30 °C, 4 °C, 45 °C, 50 °C, 55 °C, 60 °C, 65 °C, 70 °C, 75 °C, 80 °C, 85 °C, 90 °C, 95 °C.

In a third aspect, which is not claimed, an ophthalmic kit is characterized in one or more of the following items i to iii:
i. the ophthalmic kit further comprises a syringe (such as a medical syringe);
ii. the ophthalmic kit is an ophthalmic injection kit;
iii. the active pharmaceutical ingredient is at least one selected from the group consisting of dexamethasone, latanoprost, dasatinib, triamcinolone, and pharmaceutically acceptable salts thereof.

A fourth aspect, which is not claimed, relates to an ophthalmic sustained-release medicament, which comprises the ophthalmic pharmaceutical composition according to the first aspect of the present invention or the ophthalmic kit according to the third aspect of the present invention;
preferably, the ophthalmic sustained-release medicament is a sustained-release medicament for ophthalmic injection.

A fifth aspect, which is not claimed, relates to use of the ophthalmic pharmaceutical composition according to the first aspect of the present invention, the ophthalmic kit according to the third aspect of the present invention, or the ophthalmic sustained-release medicament according to the fourth aspect of the present invention in manufacture of a medicament for the prevention and/or treatment of an eye disease.

In some embodiments of the fifth aspect of the present invention, the eye disease is a disease of posterior segment of the eyeball .

In some embodiments of the fifth aspect of the present invention, the eye disease is at least one selected from the group consisting of cataract postoperative inflammation, glaucoma, uveitis, retinal vein occlusion, retinal artery occlusion, diabetic retinopathy, retinal phlebitis, proliferative vitroretinopathy, choroidal neovascularization, cystoid macular edema, age-related macular degeneration, vitreous macular adhesion, macular hole, optic neuritis, optic disc edema, optic nerve meningioma, optic nerve gliomas, retinoblastoma and choroidoblastoma.

A sixth aspect, which is not claimed, relates to a method for preventing and/or treating an eye disease, comprising a step of injecting the ophthalmic pharmaceutical composition according to the first aspect of the present invention or the ophthalmic sustained-release medicament according to the fourth aspect of the present invention into an eye.

In some embodiments of the sixth aspect, which is not claimed, the eye disease is a disease of posterior segment of the eyeball.

In some embodiments of the sixth aspect, which is not claimed, the eye disease is at least one selected from the group consisting of cataract postoperative inflammation, glaucoma, uveitis, retinal vein occlusion, retinal artery occlusion, diabetic retinopathy, retinal phlebitis, proliferative vitroretinopathy, choroidal neovascularization, cystoid macular edema, age-related macular degeneration, vitreous macular adhesion, macular hole, optic neuritis, optic disc edema, optic nerve meningioma, optic nerve gliomas, retinoblastoma and choroidoblastoma.

In some embodiments of the sixth aspect, which is not claimed, the injecting is performed into a posterior segment of the eyeball.

The term "matrix" refers to a substance that does not react with the active pharmaceutical ingredient and is conducive to play the role of the active pharmaceutical ingredient, for example, a substance having effects on administration, release, penetration and absorption of the active pharmaceutical ingredient, a substance capable of shaping the active pharmaceutical ingredient, etc.

The term "liquid matrix" refers to a matrix that is liquid at ordinary temperatures.

The term "solid matrix" refers to a matrix that is solid at ordinary temperatures.

The present invention achieves at least one of the following beneficial effects:
1. The ophthalmic pharmaceutical composition of the present invention can be injected into the eye by injection, and can continuously and slowly release the active pharmaceutical ingredient in the eye.
2. The ophthalmic pharmaceutical composition of the present invention can be degraded in the eye.
3. The stability of the ophthalmic pharmaceutical composition of the present invention is good.
4. The ophthalmic kit of the present invention can be used to prepare a medicament on site for use, thereby extending the shelf life of the medicament.

### Brief Description of the Drawings

The drawings described here are used to provide a further understanding of the present invention and constitute a part of the present application. The schematic examples of the present invention and the descriptions thereof are used to explain the present invention and do not constitute an improper limitation on the present invention. In the drawings:
Figure 1 shows a schematic diagram of in vitro release rate of the ophthalmic preparation 36 in Experimental Example 3;
Figure 2 shows a schematic diagram of in vitro release rate of the ophthalmic preparation 37 in Experimental Example 3;
Figure 3 shows a schematic diagram of in vitro release rate of the ophthalmic preparation 38 in Experimental Example 3;
Figure 4 shows a schematic diagram of in vitro release rate of the ophthalmic preparation 39 in Experimental Example 3;
Figure 5 shows a schematic diagram of in vitro release rate of the ophthalmic preparation 24 in Experimental Example 3;
Figure 6 shows a schematic diagram of intravitreal release amount of the ophthalmic preparation 36 in rabbit eye in Experimental Example 7;
Figure 7 shows a schematic diagram of intravitreal release amount of the ophthalmic preparation 37 in rabbit eye in Experimental Example 7;
Figure 8 shows a schematic diagram of intravitreal release amount of the ophthalmic preparation 38 in rabbit eye in Experimental Example 7;
Figure 9 shows a schematic diagram of intravitreal release amount of the ophthalmic preparation 39 in rabbit eye in Experimental Example 7;
Figure 10 shows a schematic diagram of intravitreal release amount of the ophthalmic preparation 24 in rabbit eye in Experimental Example 7.

### Specific Models for Carrying Out the Invention

In the following, the technical solutions in the examples of the present invention are clearly and completely described with reference to the drawings in the examples of the present invention. Obviously, the described examples are only part of examples of the present invention, but not all of the examples. The following description for at least one exemplary example is actually merely illustrative and is in no way intended to limit the invention and its application or uses. Based on the examples of the present invention, all other examples obtained by a person of ordinary skill in the art without creative efforts shall fall within the protection scope of the present invention.

The materials used in the following Preparation Examples and Comparative Examples are as follows:
PEG200: having a number average molecular weight of 190 to 210.

PEG300: having a number average molecular weight of 300.

PEG400: having a number average molecular weight of 380 to 420.

PEG600: having a number average molecular weight of 570 to 630.

Polyethylene glycol 2000: having a number average molecular weight of 1800 to 2200.

Polylactic acid-co-glycolic acid copolymer: having a weight average molecular weight of 5000 to 50,000, in which the mass ratio of polylactic acid to glycolic acid in the copolymer is 1: 3.

Polylactic acid: having a weight average molecular weight of 3,000 to 60,000.

Polyglycolic acid: having a weight average molecular weight of 5000 to 50,000.

### Preparation Examples 1 to 17: Preparation of ophthalmic preparations 1 to 17

According to the formula in Table 1, the polylactic acid-co-glycolic acid copolymer was weighed and placed into a beaker, the liquid matrix at formula amount was added thereto, and stirred at 60 °C until the polylactic acid-co-glycolic acid copolymer was completely dissolved, then the drug at formula amount was added thereto under stirring, continuously stirred until the mixture was uniform, and the mixture was filled into 1 mL glass syringes, each having a packaging volume of 200 µL. After sterilization at 121 °C for 15 minutes, the ophthalmic preparations 1 to 17 were obtained. The ophthalmic preparations 2, 18, 19, 24, 35, 40 are according to the claims.

**Table 1**

| Ophthalmic preparation | Drug | First liquid matrix | Second liquid matrix | Solid matrix | Mass ratio* |
|---|---|---|---|---|---|
| 1 | Dexamethasone | - | PEG400 | Polylactic acid-co-glycolic acid copolymer | 1: 0.1: 0.1 |
| 2 | Dexamethasone | - | PEG400 | Polylactic acid-co-glycolic acid copolymer | 1: 1: 0.5 |
| 3 | Dexamethasone | - | PEG400 | Polylactic acid-co-glycolic acid copolymer | 1: 20: 2 |
| 4 | Dexamethasone | - | Glycerol | Polylactic acid-co-glycolic acid copolymer | 1: 5: 2 |
| 5 | Dexamethasone | Dimethyl glutarate | - | Polylactic acid-co-glycolic acid copolymer | 1: 3: 0.1 |
| 6 | Dexamethasone | Diethyl succinate | - | Polylactic acid-co-glycolic acid copolymer | 1: 1: 0.5 |
| 7 | Dexamethasone | Diethyl succinate | - | Polylactic acid-co-glycolic acid copolymer | 1: 20: 2 |
| 8 | Dexamethasone | Diethyl succinate | - | Polylactic acid-co-glycolic acid copolymer | 1: 5: 2 |
| 9 | Dexamethasone | Diethyl succinate | - | Polylactic acid-co-glycolic acid copolymer | 1: 1: 1 |
| 10 | Dexamethasone | Diethyl tartarate | - | Polylactic acid-co-glycolic acid copolymer | 1: 10: 1 |
| 11 | Dexamethasone | Dimethyl glutarate | - | Polylactic acid-co-glycolic acid copolymer | 1: 3: 1 |
| 12 | Dexamethasone | Glycerol triacetate | - | Polylactic acid-co-glycolic acid copolymer | 1: 20: 2 |
| 13 | Dexamethasone | Diethyl succinate | PEG400 | Polylactic acid-co-glycolic acid copolymer | 1: 3: 7: 2 |
| 14 | Dexamethasone | Diethyl succinate | PEG400 | Polylactic acid-co-glycolic acid copolymer | 1: 8: 2: 2 |
| 15 | Dexamethasone | Dimethyl glutarate | PEG400 | Polylactic acid-co-glycolic acid copolymer | 1: 9: 1: 1 |
| 16 | Dexamethasone | Glycerol triacetate | PEG400 | Polylactic acid-co-glycolic acid copolymer | 1: 3: 1: 1 |
| 17 | Dexamethasone | Diethyl succinate | PEG600 | Polylactic acid-co-glycolic acid copolymer | 1: 3: 7: 2 |

| | | | | | |
|---|---|---|---|---|---|
| * indicates the mass ratio of the drug, the first liquid matrix or the second liquid matrix and the solid matrix, or the mass ratio of the drug, the first liquid matrix, the second liquid matrix and the solid matrix. | | | | | |

### Preparation Example 18: Preparation of ophthalmic preparation 18

50 g of polylactic acid-co-glycolic acid copolymer was weighed, put in a beaker, added 50 g of PEG400 thereto, and stirred at 60 °C until the polylactic acid-co-glycolic acid copolymer was completely dissolved, and added 20 g of dexamethasone thereto under stirring, stirred continuously until the dexamethasone and the above solution were mixed uniformly to obtain the ophthalmic preparation 18.

### Preparation Example 19: Preparation of ophthalmic preparation 19

25 g of polylactic acid-co-glycolic acid copolymer was weighed, put in a beaker, added 50 g of PEG200 thereto, and stirred at 60 °C until the polylactic acid-co-glycolic acid copolymer was completely dissolved, and added 50 g of dexamethasone thereto under stirring, stirred continuously until the dexamethasone and the above solution were mixed uniformly to obtain the ophthalmic preparation 19.

### Preparation Example 20: Preparation of ophthalmic preparation 20

100 g of polylactic acid-co-glycolic acid copolymer was weighed, put in a beaker, added 1000 g of PEG400 thereto, and stirred at 60 °C until the polylactic acid-co-glycolic acid copolymer was completely dissolved, and added 50 g of dexamethasone thereto under stirring, stirred continuously until the dexamethasone and the above solution were mixed uniformly to obtain the ophthalmic preparation 20.

### Preparation Example 21: Preparation of ophthalmic preparation 21

100 g of polylactic acid-co-glycolic acid copolymer was weighed, put in a beaker, added 250 g of PEG300 thereto, and stirred at 60 °C until the polylactic acid-co-glycolic acid copolymer was completely dissolved, and added 50 g of dexamethasone thereto under stirring, stirred continuously until the dexamethasone and the above solution were mixed uniformly to obtain the ophthalmic preparation 21.

### Preparation Example 22: Preparation of ophthalmic preparation 22

5 g of polylactic acid-co-glycolic acid copolymer was weighed, put in a beaker, added 5 g of PEG400 thereto, and stirred at 60 °C until the polylactic acid-co-glycolic acid copolymer was completely dissolved, and added 50 g of dexamethasone thereto under stirring, stirred continuously until the dexamethasone and the above solution were mixed uniformly to obtain the ophthalmic preparation 22.

### Preparation Example 23: Preparation of ophthalmic preparation 23

4950 g of polylactic acid-co-glycolic acid copolymer was weighed, put in a beaker, added 2250 g of PEG600 thereto, and stirred at 60 °C until the polylactic acid-co-glycolic acid copolymer was completely dissolved, and added 50 g of dexamethasone thereto under stirring, stirred continuously until the dexamethasone and the above solution were mixed uniformly to obtain the ophthalmic preparation 23.

### Preparation Example 24: Preparation of ophthalmic preparation 24

30 g of polylactic acid-co-glycolic acid copolymer was weighed, added 40 g of PEG400 thereto, and added 20 g of triamcinolone thereto under stirring, stirred evenly, filled into 1 ml glass syringes, each having a packaging volume of 150 µL, and sterilized at 121 °C for 15 min to obtain the ophthalmic preparation 24.

### Preparation Example 25: Preparation of ophthalmic preparation 25

25 g of polylactic acid was weighed, added 40 g of PEG400 and 10 g of PEG600 thereto, and added 50 g of gefitinib thereto under stirring, stirred evenly, filled into 1 ml glass syringes, each having a packaging volume of 200 µL, and sterilized at 121 °C for 15 min to obtain the ophthalmic preparation 25.

### Preparation Example 26: Preparation of ophthalmic preparation 26

10 g of polylactic acid-co-glycolic acid copolymer was weighed, added 10 g of PEG400 thereto, and added 5 g of sunitinib thereto under stirring, stirred evenly, filled into 0.5 ml prefilled glass syringes, each having a packaging volume of 0.15 mL to obtain the ophthalmic preparation 26.

### Preparation Example 27: Preparation of ophthalmic preparation 27

10 g of polylactic acid-co-glycolic acid copolymer was weighed, added 5 g of glycerol and 5 g of PEG600 thereto, and added 5 g of bromfenac sodium thereto under stirring, stirred evenly, filled into 1 ml glass syringes, each having a packaging volume of 200 µL, and sterilized at 121 °C for 15 min to obtain the ophthalmic preparation 27.

### Preparation Example 28: Preparation of ophthalmic preparation 28

20 g of polylactic acid-co-glycolic acid copolymer was weighed, added 15 g of propylene glycol and 25 g of PEG300 thereto, and added 10 g of timolol thereto under stirring, stirred evenly, filled into 1 ml glass syringes, each having a packaging volume of 200 µL, and sterilized at 121 °C for 15 min to obtain the ophthalmic preparation 28.

### Preparation Example 29: Preparation of ophthalmic preparation 29

10 g of polyglycolic acid was weighed, added 25 g of PEG400 thereto, stirred at 65 °C until the polyglycolic acid was completely dissolved, and added 5 g of irinotecan thereto under stirring, stirred continuously until the irinotecan and the above matrix were mixed uniformly, filled into 1 ml prefilled glass syringes by an automatic filling machine, each having a packaging volume of 0.2 mL, and sterilized at 121 °C for 15 min to obtain the ophthalmic preparation 29.

### Preparation Example 30: Preparation of ophthalmic preparation 30

10 g of polylactic acid-co-glycolic acid copolymer was weighed, added 5 g of glycerol and 10 g of PEG600 thereto, added 5 g of sirolimus thereto under stirring, stirred evenly, filled into 0.5 ml prefilled glass syringes by an automatic filling machine, each having a packaging volume of 0.15 mL, and sterilized at 121 °C for 15 min to obtain the ophthalmic preparation 30.

### Preparation Example 31: Preparation of ophthalmic preparation 31

20 g of polylactic acid-co-glycolic acid copolymer was weighed, added 30 g of glycerol triacetate thereto, added 1 g of latanoprost thereto under stirring, stirred evenly, filled into 1 ml glass syringes, and sterilized to obtain the ophthalmic preparation 31.

### Preparation Example 32: Preparation of ophthalmic preparation 32

50 g of polylactic acid-co-glycolic acid copolymer was weighed, added 100 g of glycerol triacetate thereto, added 40 g of dasatinib thereto under stirring, stirred evenly, filled into 1 ml glass syringes, and sterilized to obtain the ophthalmic preparation 32.

### Preparation Example 33: Preparation of ophthalmic preparation 33

2 g of polylactic acid was weighed, added 1 g of PEG400 thereto, added 1 g of triamcinolone thereto under stirring, stirred evenly, filled into 1 ml glass syringes, and sterilized to obtain the ophthalmic preparation 33.

### Preparation Example 34: Preparation of ophthalmic preparation 34

4 g of polylactic acid-co-glycolic acid copolymer was weighed, added 4 g of PEG400, 0.5 g of glycerol and 0.5 g of propylene glycol thereto, added 4 g of paclitaxel thereto under stirring, stirred evenly, filled into 1 ml glass syringes, and sterilized to obtain the ophthalmic preparation 34.

### Preparation Example 35: Preparation of ophthalmic preparation 35

6 g of polylactic acid-co-glycolic acid copolymer was weighed, added 9 g of PEG400 thereto, added 6 g of dexamethasone thereto under stirring, stirred evenly, filled into 1 ml glass syringes, and sterilized to obtain the ophthalmic preparation 35.

### Preparation Examples 36 to 39: Preparation of ophthalmic preparations 36 to 39

30 g of polylactic acid-co-glycolic acid copolymer was weighed, added 10 g of PEG400 thereto, then added 30 g of diethyl succinate, 30 g of diethyl tartarate, 30 g of dimethyl glutarate or 30 g of glycerol triacetate thereto respectively, added 30 g of triamcinolone thereto under stirring, stirred evenly, filled into 1 ml glass syringes, each having a packaging volume of 150 µL, and sterilized at 121 °C for 15 min to obtain the ophthalmic preparations 36 to 39 respectively.

### Preparation Example 40: Preparation of ophthalmic preparation 40

30 g of polylactic acid-co-glycolic acid copolymer was weighed, added 40 g of PEG400 thereto, added 30 g of triamcinolone thereto under stirring, stirred evenly, filled into prefilled glass syringes to obtain the ophthalmic preparation 40.

### Preparation Examples 41 to 44: Preparation of ophthalmic preparations 41 to 44

40 g of polylactic acid-co-glycolic acid copolymer, 40 g of polylactic acid, 40 g of polyglycolic acid and 40 g of polyethylene glycol 2000 were weighed respectively, added 40 g of PEG400 thereto respectively, then added 20 g of dexamethasone thereto respectively under stirring, stirred evenly, filled into prefilled glass syringes to obtain the ophthalmic preparations 41 to 44.

### Experimental Example 1: Observation of the status of ophthalmic preparations in rabbit eye vitreum or rabbit eye anterior chamber

(1) 50 µL of the ophthalmic preparations 1 to 17 and 33 to 35 were injected into the rabbit eye vitreum respectively, and the status of the ophthalmic preparations in the rabbit eye vitreum was observed.

It was found that the ophthalmic preparations 1 to 17 aggregated in the rabbit eye vitreum without dispersing, while the ophthalmic preparations 33 to 35 automatically aggregated into a spherical shape in the rabbit eye vitreum.

(2) 20 µL of the ophthalmic preparations 26 and 30 were injected into the rabbit eye anterior chamber respectively, and the status of the preparations in the rabbit eye anterior chamber was observed.

It was found that the ophthalmic preparations 26 and 30 automatically aggregated into a spherical shape in the rabbit eye anterior chamber.

### Experimental Example 2: Observation of the status of ophthalmic preparations in sodium chloride solution or phosphate buffer solution

(1) 50 µL of the ophthalmic preparations 18 to 23 and 36 to 39 were injected respectively into beakers with 0.9% sodium chloride solution at pH = 7.4 and osmotic pressure = 292 mOsmol/kg, and the status of each in sodium chloride solution was observed.

It was found that the ophthalmic preparations 18 to 23 and 36 to 39 automatically aggregated into a spherical shape in sodium chloride solution and deposited on the bottom of the beakers.

(2) 100 µL of the ophthalmic preparations 24 to 25, 27 to 28, and 31 to 32 were injected respectively into breakers with phosphate buffer solution at pH = 7.4 and osmotic pressure = 292 mOsmol/kg (the pH of 50 mmol/L potassium dihydrogen phosphate was adjusted to 7.4 with hydroxide sodium ), and the status of each in phosphate buffer solution was observed.

It was found that the ophthalmic preparations 24 to 25, 27 to 28, and 31 to 32 automatically aggregated into a spherical shape in phosphate buffer solution and deposited on the bottom of the beakers.

### Experimental Example 3: Investigation of in vitro release rate of ophthalmic preparations

50 µL of the ophthalmic preparation 24 and the ophthalmic preparations 36 to 39 were taken and injected into breakers with 200 mL of release medium (sodium chloride solution, containing 0.01% benzalkonium chloride, pH = 7.4, osmotic pressure = 292 mOsmol/kg), respectively. The beakers were placed in a gas bath oscillator, the temperature was controlled at 37 ± 0.5 °C, 10 mL of release medium was taken at different times and an equal amount of release medium was supplemented. The release medium was filtered through a 0.22 µm microporous filter membrane, and the triamcinolone content in the release medium was determined by a high performance liquid chromatography (HPLC) method.

Chromatographic conditions: Ultimate, XB-C18 column (4.6 × 250 mm, 5 µm); mobile phase: methanol-water (52.5: 47.5, v/v); detection wavelength: 240 nm; column temperature: 30 °C; flow rate: 1.0 mL·min⁻¹; injection volume: 20 µL.

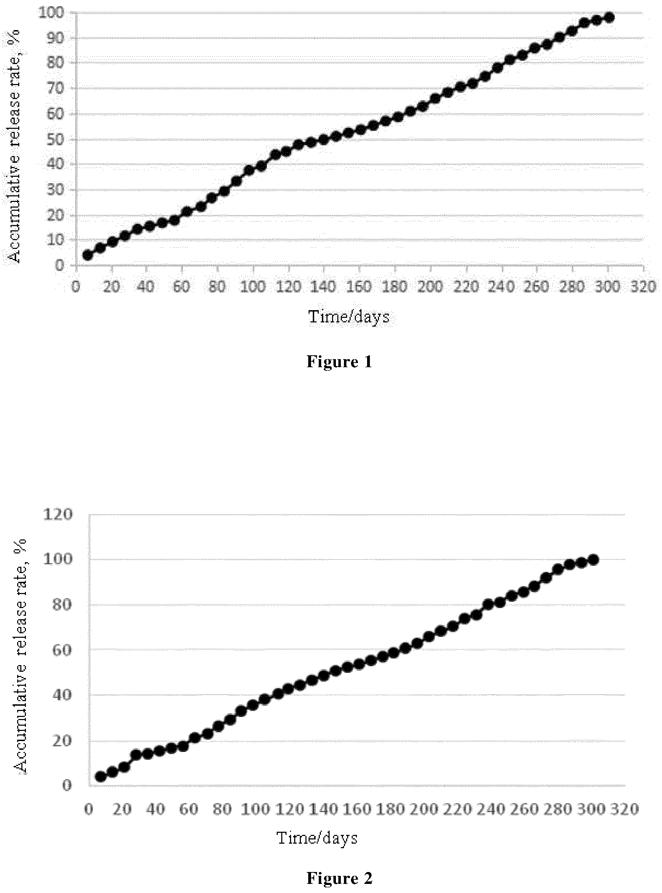

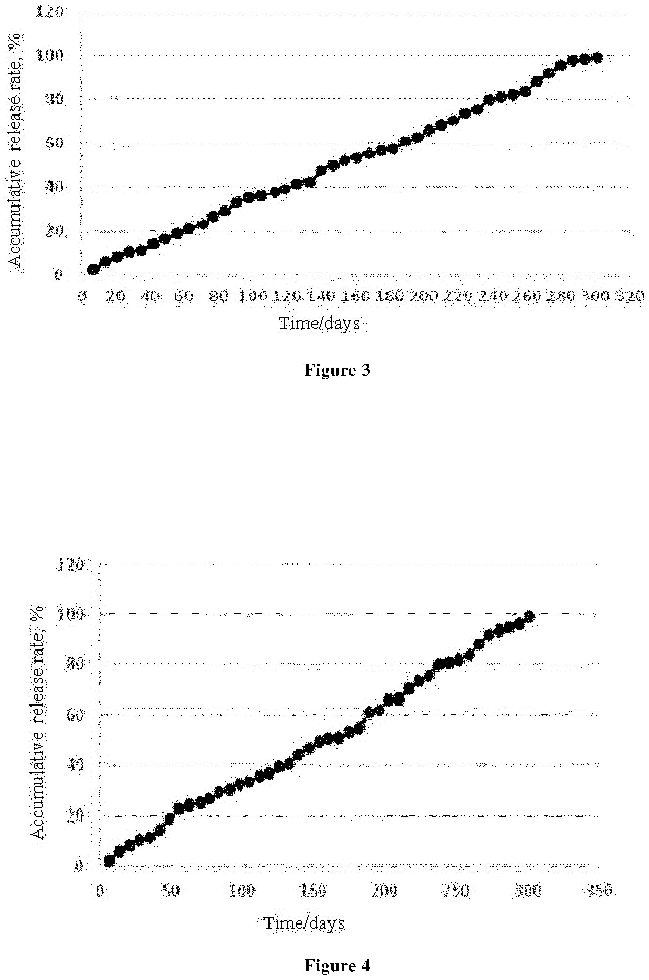

The cumulative release amount Rn% of the drug was calculated according to the following formula. The results are shown in Figures 1 to 5. $Rn% = \left({C}_{n}{V}_{0}+{\sum }_{n - 1}^{n} {C}_{n - 1} V\right) / {M}_{t} \times 100 %$ wherein, *V₀* is the release medium volume, *Cₙ* is the drug concentration in the n^{th} sampling, V is the sampling volume, and *Mₜ* is the total drug concentration.

Comparing the release results in Figures 1 to 5, it could be found that when using PEG400 together with diethyl succinate, diethyl tartrate, dimethyl glutarate, or glycerol triacetate as the liquid matrix, the sustained-release rate of the ophthalmic preparation was reduced, the sustained-release effect of a single administration could last about 1 year.

When the ophthalmic preparations 31 to 32 were used for the in vitro release test, the sustained-release effect of a single administration could last 200 days.

### Experimental Example 4: Observation of appearance change of ophthalmic preparations after being injected into rabbit eye anterior chamber

25 µL of the ophthalmic preparations 31 to 32, 36 to 39 and 40 were injected into the rabbit eye anterior chamber respectively, and the changes of the preparations in the rabbit eyes were observed every other day with a hand-held slit lamp.

The results showed that:
After injection, the ophthalmic preparations 31 to 32 aggregated into spherical milky white solid particles and gradually subsided in the lower part of the anterior chamber. With the extension of time, the volume of solid particles gradually decreased. On the 80^{th} to 85^{th} days, the solid particles completely disappeared.

After injection, the ophthalmic preparations 36 to 39 aggregated into spherical milky white solid particles and gradually subsided in the lower part of the anterior chamber. With the extension of time, the volume of solid particles gradually decreased. On the 100^{th} day, the solid particles completely disappeared.

After injection, the ophthalmic preparation 40 aggregated into spherical milky white solid particles and gradually subsided in the lower part of the anterior chamber. With the extension of time, the volume of solid particles gradually decreased, and the solid particles completely disappeared on the 92^{nd} to 97^{th} days.

These observation results indicated that the ophthalmic preparations prepared by the above method could be slowly degraded in the living body so as to continuously release the active ingredient.

### Experimental Example 5: Observation of the appearance change of ophthalmic preparations after being injected into rabbit eye vitreum

25 µL of the ophthalmic preparations 31 to 32, 36 to 39 and 40 were injected into the rabbit eye vitreum respectively, and the changes of the preparations in the rabbit eyes were observed every other day with a handheld slit lamp.

The results showed that:
After injection, the ophthalmic preparations 31 to 32 aggregated into spherical milky white solid particles and remained at the injection site. With the extension of time, the volume of solid particles gradually decreased. On the 180^{th} day, the solid particles completely disappeared.

After injection, the ophthalmic preparations 36 to 39 aggregated into spherical milky white solid particles and remained at the injection site. With the extension of time, the volume of solid particles gradually decreased. On the 300^{th} day, the solid particles completely disappeared.

After injection, the ophthalmic preparation 40 aggregated into spherical milky white solid particles and remained at the injection site. With the extension of time, the volume of solid particles gradually decreased, and the solid particles completely disappeared during the period of the 73^{rd} to 75^{th} days.

These observation results indicated that the ophthalmic preparations of the present invention could be slowly degraded in the living body so as to continuously release the active ingredient.

### Experimental Example 6: Observation of the status change of ophthalmic preparations after being injected into rabbit eye Tenon capsule

100 µL of the ophthalmic preparations 31 to 32, 36 to 39 and 40 were injected into the Tenon capsules of 60 rabbits' right eyes respectivel. 5 animals were sacrificed and dissected each month, and the state of the preparations in the Tenon capsules was observed.

The results showed that:
After injection of the ophthalmic preparations 31 to 32, the volume of the preparations in the Tenon capsules of rabbit eyes gradually decreased over time; among the 5 rabbits dissected in the 8^{th} month, the preparations in the Tenon capsules of 2 rabbits had completely disappeared; and among the 5 rabbits dissected in the 9^{th} month, the preparations in the Tenon capsules of 3 rabbits had completely disappeared, and a very small amount of the preparations remained in the Tenon capsules of the other 2 rabbits.

After injection of the ophthalmic preparations 36 to 39, the volume of the preparations in the Tenon capsules of rabbit eyes gradually decreased over time; among the 5 rabbits dissected in the 8^{th} month, the preparation in the Tenon capsule of 0 rabbit had completely disappeared; and among the 5 rabbits dissected in the 9^{th} month, the preparations in Tenon capsules of 2 rabbits had completely disappeared, and a very small amount of the preparations remained in the Tenon capsules of the other 3 rabbits.

After injection of the ophthalmic preparation 40, the volume of the preparations in the Tenon capsules of rabbit eyes gradually decreased over time; among the 5 rabbits dissected in the 8^{th} month, the preparation in the Tenon capsule of 1 rabbit had completely disappeared; among the 5 rabbits dissected in the 9^{th} month, the preparations in the Tenon capsules of 4 rabbits had completely disappeared, and a very small amount of the preparation remained in the Tenon capsule of the other 1 rabbit.

### Experimental Example 7: Observation of the release behavior of ophthalmic preparation in rabbit eye vitreum after single injection

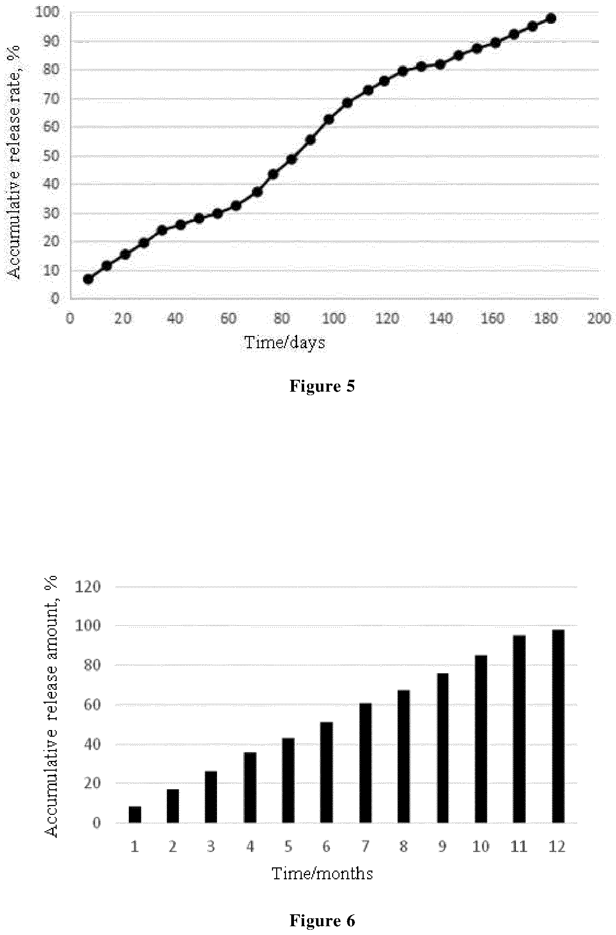

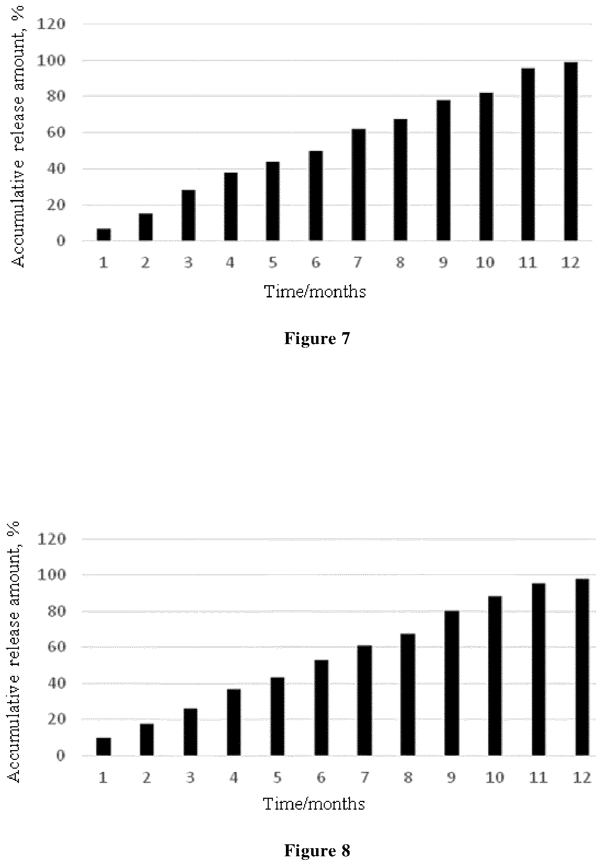

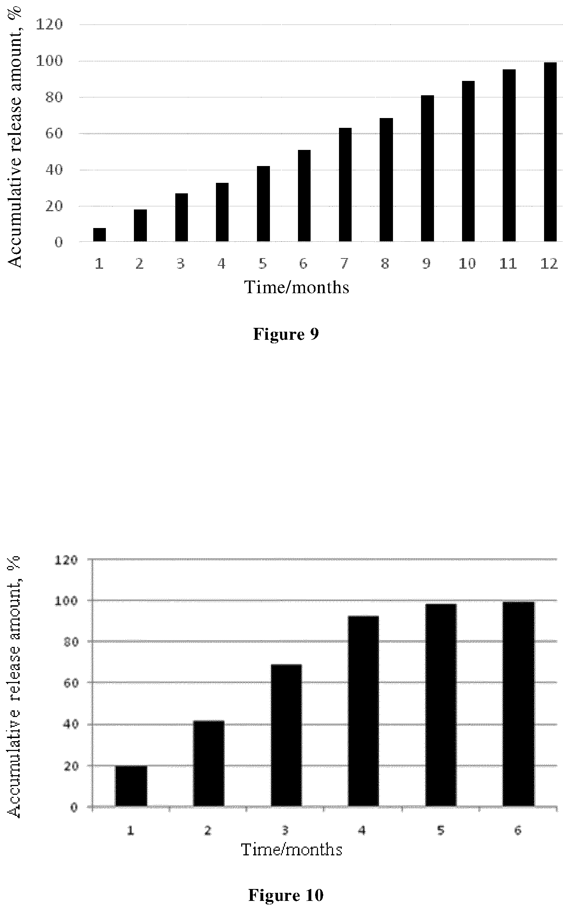

(1) 48 New Zealand rabbits were taken, randomly grouped, 4 rabbits per group. The right eyes of the rabbits in each group were injected with 50 µL of the ophthalmic preparations 36 to 39 respectively, the animals of one group were executed in each of the 1^{st}, 2^{nd}, 3^{rd} 4^{th}, 5^{th}, 6^{th}, 7^{th}, 8^{th}, 9^{th}, 10^{th}, 11^{th}, 12^{th} months after the injection, and the unreleased preparations were removed from the vitreum. The triamcinolone contents in the preparations were determined by HPLC method, and the cumulative release amounts were calculated. The results were shown in Figures 6 to 9.
(2) 36 New Zealand rabbits were taken, randomly grouped, 6 rabbits per group. The right eyes of the rabbits in each group were injected with 50 µL of the ophthalmic preparation 24, the animals of one group were executed in each of the 1^{st}, 2^{nd}, 3^{rd} 4^{th}, 5^{th}, 6^{th}, 7^{th}, 8^{th}, 9^{th}, 10^{th}, 11^{th}, 12^{th} months after the injection, and the unreleased preparations were removed from the vitreum. The triamcinolone contents in the preparations were determined by HPLC method, and the cumulative release amounts were calculated. The results were shown in Figure 10.

According to Figures 6 to 10, it could be seen that the sustained-release time of the ophthalmic preparations of the present invention in the rabbit eye vitreum was as long as 12 months, while the sustained-release time of the ophthalmic preparation 24 in the rabbit eye vitreum was only 6 months.

Therefore, the ophthalmic preparations of the present invention could significantly prolong the sustained-release time in the vitreum and maintain the drug efficacy for a long time.

## Claims

1. An ophthalmic sustained-release drug delivery system, consisting of the following components:
a solid matrix having a biocompatibility
a liquid matrix having a biocompatibility
an active pharmaceutical ingredient;
wherein,
the solid matrix is polylactic acid-co-glycolic acid copolymer;
the liquid matrix is polyethylene glycol,
the active pharmaceutical ingredient is at least one selected from the group consisting of dexamethasone, latanoprost, dasatinib, triamcinolone, and pharmaceutically acceptable salts thereof,
the weight ratio of the active pharmaceutical ingredient, the liquid matrix, and the polylactic acid-co-glycolic acid copolymer is 1:(1-2):(0.1-2).

2. The ophthalmic sustained-release drug delivery system according to Claim 1, which is an ophthalmic pharmaceutical injection preparation.

## Patentansprüche

1. Ein ophthalmisches Wirkstofffreisetzungssystem mit verzögerter Freisetzung, bestehend aus den folgenden
Komponenten:
einer festen Matrix mit einer Biokompatibilität
einer flüssigen Matrix mit einer Biokompatibilität
einem pharmazeutischen Wirkstoff;
wobei,
die feste Matrix Poly (Milchsäure-co-Glykolsäure)-Copolymer ist;
die flüssige Matrix Polyethylenglykol ist,
der pharmazeutische Wirkstoff mindestens einer ist, ausgewählt aus der Gruppe bestehend aus Dexamethason, Latanoprost, Dasatinib, Triamcinolon und pharmazeutisch verträglichen davon gebildeten Salzen,
das Gewichtsverhältnis des pharmazeutischen Wirkstoffs, der flüssigen Matrix und des Poly (Milchsäure-co-Glykolsäure)-Copolymers 1:(1-2):(0,1-2) ist.

2. Das ophthalmische Wirkstofffreisetzungssystem mit verzögerter Freisetzung gemäß Anspruch 1, wobei
es eine ophthalmische pharmazeutische Injektionspräparation ist.

## Revendications

1. Un système d'administration ophtalmique à libération prolongée, constitué des composants suivants :
une matrice solide présentant une biocompatibilité
une matrice liquide présentant une biocompatibilité
un principe actif pharmaceutique ;
dans lequel,
la matrice solide est un copolymère d'acide polylactique-co-acide glycolique ;
la matrice liquide est du polyéthylène glycol,
le principe actif pharmaceutique est au moins un élément choisi dans le groupe
constitué de dexaméthasone, de latanoprost, de dasatinib, de triamcinolone, et de leurs sels pharmaceutiquement
acceptables,
le rapport pondéral du principe actif pharmaceutique, de la matrice liquide, et du
copolymère d'acide polylactique-co-acide glycolique est de 1:(1-2):(0.1-2).

2. Le système d'administration ophtalmique à libération prolongée selon la revendication 1, qui
est une préparation injectable pharmaceutique ophtalmique.
